(19) Europäisches Patentamt
European Patent Office
Office européen des brevets

(11) Numéro de publication: **0 041 891**
**A1**

# (12) DEMANDE DE BREVET EUROPEEN

(21) Numéro de dépôt: 81400889.2

(22) Date de dépôt: 04.06.81

(51) Int. Cl.³: C 07 D 491/04
A 61 K 31/495
//(C07D491/04, 307/00, 221/00)

(30) Priorité: 05.06.80 FR 8012553

(43) Date de publication de la demande:
16.12.81 Bulletin 81/50

(84) Etats contractants désignés:
AT BE CH DE FR GB IT LI LU NL SE

(71) Demandeur: Centre Européen de Recherches Mauvernay (CERM)
Route de Marsat
F-63201 Riom(FR)

(72) Inventeur: Busch, Norbert
Le Bouquet
Loubeyrat F-63410 Manzat(FR)

(72) Inventeur: Combourieu, Michel
6, rue du Mont Mouchet
F-15000 Aurillac(FR)

(72) Inventeur: Bernet, Yvon
11, Cité des Landes
Reilhac F-15250 Jussac(FR)

(74) Mandataire: Therond, Gérard Raymond
C.E.R.M Route de Marsat
F-63201 Riom(FR)

## (54) 4-(1-pipérazinyl)-furo (3,2-c) pyridines substituées, leurs procédés d'obtention et leur application en thérapeutique.

(57) Composés répondant à la formule:

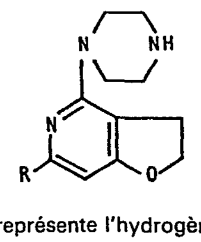

dans laquelle R représente l'hydrogène ou un radical alkyle ayant 1 à 6 atomes de carbone, et leurs sels pharmaceutiquement acceptables.

Application comme antidépresseur.

EP 0 041 891 A1

La présente invention concerne de nouvelles 4-(1-piperazinyl)-furo [3,2-c] pyridines substituées, leurs procédés de préparation, et les compositions pharmaceutiques les contenant sous forme de base libre ou de sel pharmaceutiquement acceptable.

La présente invention concerne plus particulièrement les composés de formule générale I

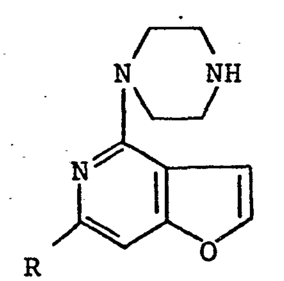

(I)

et leurs sels pharmaceutiquement acceptables dans laquelle R représente l'hydrogène ou un radical alkyle inférieur ayant 1 à 6 atomes de carbone.

Selon une réalisation préférée R représente un radical alkyle ayant 1 à 4 atomes de carbone, et particulièrement le radical méthyle.

Les composés de formule I peuvent être obtenus par condensation de la pipérazine avec une 4-halo-furo [3,2-c] pyridine substituée (Formule II).

Cette dernière matière première peut être préparée par exemple à partir d'une 4-hydroxy-2-oxo-1,2-dihydro pyridine substituée conformément au schéma réactionnel suivant :

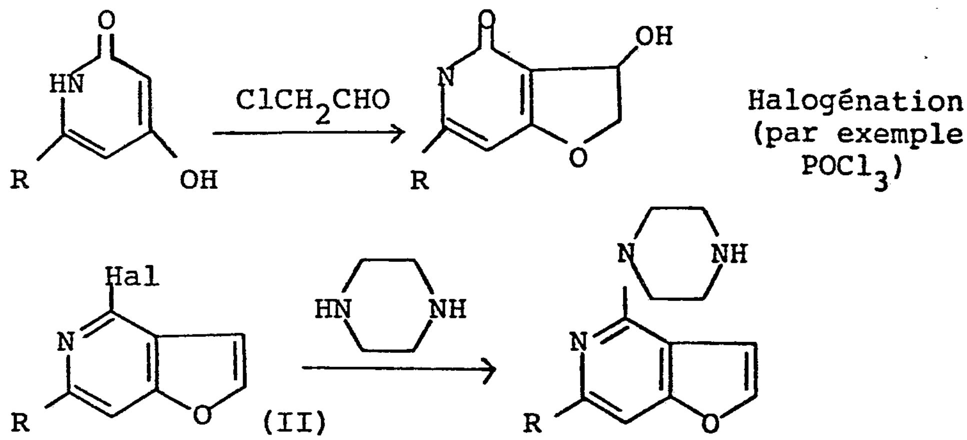


(II)

(Hal = halogène)

La condensation du composé II avec la pipérazine s'effectue en utilisant la pipérazine en excès, de préférence en chauffant le mélange à reflux dans un solvant convenable tel que l'éther mono-méthylique de l'éthylène glycol.

Pour obtenir un sel des composés de formule I, on fait agir la base libre avec un acide organique ou inorganique tels que les acides HCl, HBr, $H_3PO_4$, ou les acides acétique, maléique, fumarique, tartrique, etc...

Les composés selon l'invention et leurs sels pharmaceutiquement acceptables possèdent d'intéressantes activités psychopharmacologiques, en particulier une activité antidépressive.

Les composés de formule I, associés aux excipients pharmaceutiques habituels peuvent être administrés par voie enthérale ou parenthérale, de préférence à une dose journalière comprise entre 0,1 et 50 mg par kilo de poids corporel, et plus spécialement à une dose comprise entre 1 et 10 mg par kilo de poids corporel. La dose journalière préférée chez l'homme est d'environ 50 à 100 mg.

L'activité psychopharmacologique et spécialement l'activité antidépressive des composés de formule I (dans lesquels R représente l'hydrogène ou le radical méthyle) est illustrée par le résultat des tests suivants :

A - Antagonisme du ptosis réserpinique chez la souris

Le produit étudié (ou le véhicule seul - gomme arabique et eau 1/1 - pour le lot témoin) est administré par intubation oesophagienne à des souris de sexe mâle, de souche OF1 IFFA CREDO de poids moyen 22 $\pm$ 1 g, puis 60 minutes après traitement, tous les animaux reçoivent par voie intrapéritonéale 1 $mg.kg^{-1}$ de Réserpine base.

Le composé de l'exemple 1 et la quipazine prise comme référence, ont été administrés à raison de 40 $mg.kg^{-1}$ et le composé de l'exemple 2 à raison de 10 $mg.kg^{-1}$.

L'appréciation du ptosis (oeil gauche) est donnée selon la cotation de RUBIN et Coll. [J. Pharmacol. Exp. Ther. 120, 125-136 (1957)] 3 heures après l'administration de Réserpine.

B - Potentialisation des stéréotypies induites par l'amphétamine

Ce test est effectué sur des rats SPRAGUE-DAWLEY de souche OFA, provenant de l'élevage IFFA CREDO et d'un poids moyen de 150 ± 10 g.

Le protocole mis en oeuvre est celui préconisé par SIMON et CHERMAT /J. Pharmacol. (Paris) 3 (2) 235-238 (1972)/. Le produit étudié (ou le véhicule seul pour le lot témoin) est administré par voie intrapéritonéale à raison de 5 $mg.kg^{-1}$.

Les résultats expriment le score global sur 3 heures.

C - Antagonisme du comportement " muricide " chez le rat LONG-EVANS

Les animaux utilisés sont des rats de sexe mâle LONG-EVANS de souche pie-noire, en provenance de l'élevage IFFA CREDO, dont la période d'isolement dans des cages individuelles est supérieure à 6 mois.

L'administration des composés (ou du véhicule pour les animaux de groupe de contrôle) est effectuée par voie intrapéritonéale à raison de 5 $mg.kg^{-1}$, 60 minutes avant la mise en présence d'une souris avec les rats isolés ($n \geq 8$).

Le comportement classiquement décrit comme " muricide " /VALZELLI L. - Actualités Pharmacologiques, 24, 133-152, (1971)/ est positif si le rat tue la souris en un temps inférieur ou égal à 3 minutes.

Les résultats sont exprimés en pourcentage de diminution par rapport à celui du groupe des animaux de contrôle.

Les résultats observés dans ces trois tests sont résumés dans le tableau ci-après comparativement à la quipazine.

Dans ce tableau, on indique en outre une estimation de la DL 50 des composés par voie orale chez la souris.

| TESTS | EXEMPLE 1 ($R=CH_3$) | EXEMPLE 2 (R=H) | QUIPAZINE |
|---|---|---|---|
| A-Activité anti-réserpinique vis-à-vis du ptosis | 13 / 23 | 15 / 22 | 14 / 23 |
| B-Potentialisation des stéréotypies induites par l'amphétamine | 118/95 : 24,2 % | 129/114 : 13,2% | Inactif même à 20 I P |
| C-Antagonisme du comportement muricide | 90 % | 74 % | 64,7 % |
| Evaluation DL 50 | 250 $mg.kg^{-1}$ | 200 $mg.kg^{-1}$ | [233-376] $mg.kg^{-1}$ |

La synthèse des composés de formule I est illustrée plus en détails dans les exemples qui suivent.

Préparation des matières premières de formule II

Première étape : 6-methyl-4-oxo 3-hydroxy dihydro 2,3 furo /3,2-c/ pyridine

Dans un ballon contenant 83 g (0,6 M) de carbonate de potassium, on a ajouté sous agitation à température ambiante 50 g (0,4 M) de 6-methyl-4-hydroxy-2-oxo-1,2-dihydro pyridine. Après avoir laissé le mélange sous agitation pendant environ 30 minutes, on a introduit en une seule fois sous violente agitation 0,6 M de chloracétaldéhyde, soit 96 g de la solution

commerciale à 50 % dans l'eau. Après avoir laissé la réaction se poursuivre environ 30 minutes sous agitation en maintenant la température entre 25-30°C, on a refroidi et séparé par essorage le produit attendu. Après séchage, le produit a été recristallisé dans un mélange benzène éthanol (1/1) pour donner 49,5 g de produit se présentant sous forme d'aiguilles incolores ayant pour point de fusion F = 265°.

Deuxième étape : 6-methyl-4-chloro-furo [3,2-c]pyridine

Dans un ballon contenant 150 ml d'oxychlorure de phosphore rectifié, préalablement porté à 40°C, on a introduit par petites portions 9 g (0,06 M) du composé précédemment préparé. Cette première phase de la réaction correspond à la déshydratation en furopyridone. On a ensuite chauffé le mélange à reflux pendant 1 heure 30, puis éliminé l'excès de $POCl_3$ sous pression réduite. Le résidu a ensuite été versé dans 200 ml d'eau glacée, la solution résultante filtrée, neutralisée à pH 7 par de la soude et le produit formé extrait au chloroforme. Après séchage sur sulfate de sodium et évaporation du solvant, le produit formé a été distillé sous pression réduite ($Eb_{17}$ = 125-127°). Le produit a ensuite été recristallisé dans le minimum de pentane pour donner 6,9 g de paillettes incolores F = 65-66°.

EXEMPLE 1

6-methyl-4-(1-piperazinyl)-furo [3,2-c] pyridine

Dans 50 ml d'éther monométhylique de l'éthylène glycol, on a ajouté 8,4 g (0,05 M) du dérivé chloré précédemment obtenu et 12,9 g (0,15 M) de pipérazine, puis chauffé le mélange à reflux pendant 72 heures.

Le solvant a ensuite été évaporé et le résidu repris par l'eau. Après élimination par essorage d'une petite fraction insoluble, le milieu a été alcalinisé, puis le produit extrait au chloroforme. Après évaporation du solvant et distillation, on a obtenu 8,8 g de produit du titre ayant pour point d'ébullition $Eb_{13}$ = 200-205°.

Pour obtenir un sel cristallisé de ce composé, on a dissous 3 g de la base ainsi obtenue dans l'acétone, puis ajouté 1,9 g d'acide maléique également en solution dans l'acétone. Après chauffage à ébullition, le mélange a été refroidi, le précipité essoré, lavé à l'acétone puis séché. On a ainsi obtenu 4,3 g d'une poudre de couleur crème ayant pour point de fusion F = 180°C et pour analyse élémentaire :

| | C % | H % | N % |
|---|---|---|---|
| Calculé | 57,65 | 5,75 | 12,61 |
| Trouvé | 56,95 | 5,79 | 12,61 |

EXEMPLE 2

4-(1-piperazinyl)-furo [3,2-c] pyridine

En procédant comme indiqué dans l'exemple 1, mais en partant de 8 g (0,052 M) de 4-chloro-furo [3,2-c] pyridine et de 22,4 g (0,25 M) de piperazine, on a obtenu 4,1 g de produit du titre sous forme d'huile ayant pour point d'ébullition $Eb_{20}$ = 195-200°.

De la même façon, on a également obtenu le composé de l'invention sous forme de sel cristallisé, le monomaléate hydraté (1/4 $H_2O$ par mole) ayant pour point de fusion F = 295°C et pour analyse élémentaire :

| | C % | H % | N % |
|---|---|---|---|
| Calculé | 55,64 | 5,41 | 12,98 |
| Trouvé | 55,21 | 5,28 | 13,13 |

REVENDICATIONS

1 - 4-(1-piperazinyl)-furo /3,2-c/ pyridines répondant à la formule :

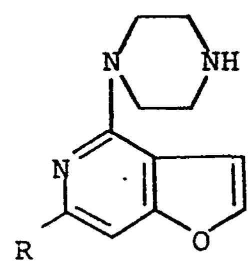

(I)

dans laquelle R représente l'hydrogène ou un radical alkyle ayant 1 à 6 atomes de carbone et leurs sels pharmaceutiquement acceptables.

2 - Composé selon la revendication 1 caractérisé en ce que R représente le radical méthyle.

3 - Procédé pour l'obtention d'un composé selon la revendication 1 caractérisé en ce qu'on condense la pipérazine en excès en milieu solvant sur une 4-halo-furo (3,2-c) pyridine, en transformant en outre si nécessaire le composé de formule I ainsi obtenu en sel pharmaceutiquement acceptable.

4 - Composition pharmaceutique contenant à titre de principe actif un composé selon les revendications 1 ou 2, associé avec un ou plusieurs excipients pharmaceutiques habituels.

5 - Application des composés selon les revendications 1 ou 2 en tant que produits antidépresseurs.

**0041891**

Numéro de la demande

Office européen des brevets

## RAPPORT DE RECHERCHE EUROPEENNE

EP 81 40 0889

| Catégorie | DOCUMENTS CONSIDERES COMME PERTINENTS — Citation du document avec indication, en cas de besoin, des parties pertinentes | Revendication concernée | CLASSEMENT DE LA DEMANDE (Int. Cl.³) |
|---|---|---|---|
| | CHIMIE THERAPEUTIQUE, vol. VIII, no. 5, septembre-octobre 1973 CHATENAY-MALABRY (FR)<br>E. BISAGNI et al.:"Aza-indoles-II. Nouveaux dérivés aza-5 indoliques et leurs propriétés pharmacologiques"<br>pages 559-566<br>* tableaux B,E et F, composé 17 *<br>-- | 1,4 | C 07 D 491/04<br>A 61 K 31/495//<br>(C 07 D 491/04<br>307/00<br>221/00) |
| | BULLETIN DE LA SOCIETE CHIMIQUE DE FRANCE, no. 5, mai 1971 PARIS (FR)<br>J.-D. BOURZAT et al.:"Furannes et pyrroles disubstitués en 2,3.X. Synthèse de furo(3,2-c)pyridines à partir du formyl-2 carbéthoxy-3 furanne"<br>pages 1727-30<br>* page 1728, schéma réactionnel et composés 13,15 et 16 *<br>------ | 1,3 | DOMAINES TECHNIQUES RECHERCHES (Int. Cl.³)<br>C 07 D 491/04<br>A 61 K 31/495// |

CATEGORIE DES DOCUMENTS CITES

X: particulièrement pertinent
A: arrière-plan technologique
O: divulgation non-écrite
P: document intercalaire
T: théorie ou principe à la base de l'invention
E: demande faisant interférence
D: document cité dans la demande
L: document cité pour d'autres raisons
&: membre de la même famille, document correspondant

X Le présent rapport de recherche a été établi pour toutes les revendications

| Lieu de la recherche | Date d'achèvement de la recherche | Examinateur |
|---|---|---|
| La Haye | 14-08-1981 | ALFARO |

OEB Form 1503.1 06.78